# DEMANDE DE BREVET EUROPEEN

(11) **EP 4 850 594 A2**
(43) Date de publication de la demande: **09.12.2026**
(21) Numéro de dépôt: 26205750.8
(22) Date de dépôt: 17.10.2024
(51) Int. Cl.: A61B 5/00

(54) **PROCÉDÉ ET DISPOSITIF POUR LA SPECTROSCOPIE PAR RÉFLECTANCE DIFFUSE COMPRENANT UNE MODULATION EN INTENSITÉ ET/OU EN FRÉQUENCE OPTIQUE DU RAYONNEMENT OPTIQUE**

(30) Priorité: 17.10.2023 FR 2311219
(62) Numéro(s) de document de la (des) demande(s) initiale(s) en application de l'article 76 CBE: 24207295.7 / 4 541 264
(71) Demandeur: Commissariat à l'Energie Atomique et aux Energies Alternatives, 75015 Paris (FR)
(72) Inventeur: ANDRE, Luc, 38054 Grenoble (FR); KOENIG, Anne, 38054 Grenoble (FR); BARDET, Mélina, 38054 Grenoble (FR)
(74) Mandataire: Ipsilon

(57) **Abrégé**

L'invention concerne un procédé de mesure, par spectroscopie par réflectance diffuse, du coefficient d'atténuation d'une partie d'un corps (1) diffusant et/ou absorbant, le procédé comprenant les étapes suivantes :
a) émission d'un rayonnement optique dont l'intensité et/ou la fréquence optique sont modulées, au moins une partie du rayonnement optique, dénommée signal de sonde, irradiant le corps,
b) réception d'une partie du signal de sonde diffusée et réfléchie par le corps, dénommée signal rétrodiffusé, et mesure de la longueur de parcours d du signal rétrodiffusé,
c) mesure de la réflectance *R* de la partie du corps traversée par le signal rétrodiffusé,
d) calcul du coefficient d'atténuation *µ* à partir de la longueur de parcours *d* et de la réflectance *R* mesurées.

## Description

### Domaine technique

La présente invention concerne le domaine des capteurs optiques pour la spectroscopie par réflectance diffuse, aussi connue sous l'abréviation « DRS ».

### Technique antérieure

La spectroscopie par réflectance diffuse (DRS) est une technique de mesure non-invasive permettant l'étude de la structure et/ou de la composition d'un corps diffusant. Elle est notamment utilisée pour mesurer la concentration de chromophores dans un tissu biologique, par exemple pour mesurer le taux d'oxygénation et/ou le taux d'hydratation et/ou de glycémie des tissus cutanés.

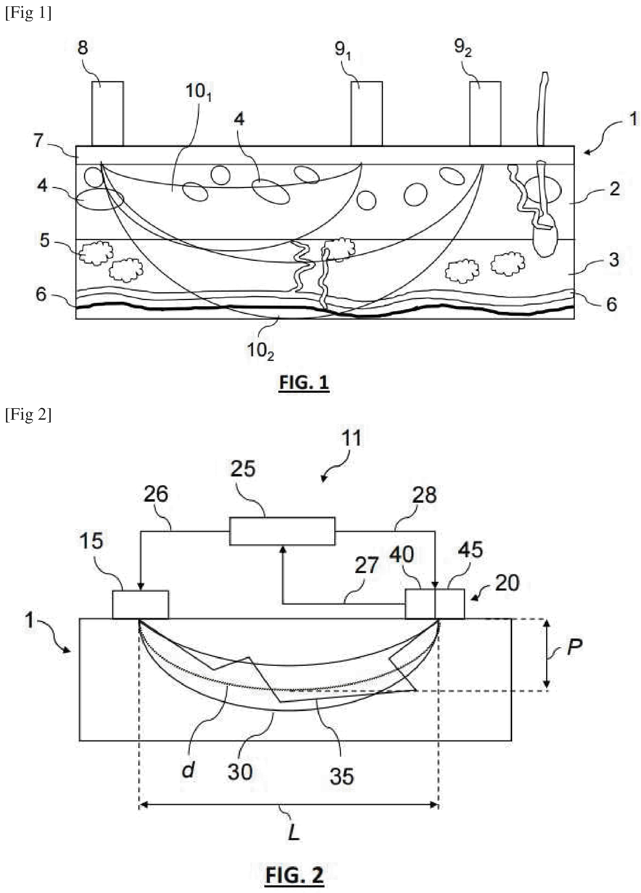

On a illustré sur la figure 1 un exemple de mise en œuvre de DRS selon l'art antérieur pour l'étude d'un tissu cutané 1. Le tissu cutané 1 comprend un épiderme 2 et un derme 3 recouvert par l'épiderme 2. L'épiderme 2 comprend des cellules d'épiderme 4 et le derme 3 comprend des cellules de derme 5 et des vaisseaux sanguins 6. Le tissu cutané 1 comprend également une couche cornée 7 composée de cellules mortes recouvrant l'épiderme 2.

Un dispositif pour la mise en œuvre de la DRS met généralement en œuvre une source lumineuse 8 émettant un rayonnement optique de longueur d'onde comprise entre 400 nm et 1700 nm dans le tissu cutané 1. Il comporte en outre des capteurs optiques 9₁, 9₂ pour détecter une partie rétrodiffusée 10₁, ou 10₂, du rayonnement optique qui a interagi avec le tissu cutané 1.

Au cours de leur trajet au sein du tissu cutané 1, les photons du rayonnement optique sont absorbés, défléchis et/ou diffusés par les constituants 4-6 absorbants ou diffusants du tissu cutané 1. Les constituants 4-6 absorbants sont notamment les chromophores, par exemple l'eau, certains lipides, la mélanine, l'hémoglobine ou le glucose. Les constituants 4-6 diffusants sont notamment les mélanosomes, les cellules sanguines, le collagène, la kératine et certains lipides. Seule une partie 10₁, 10₂, des photons est rétrodiffusée, c'est-à-dire qu'elle émerge par réflexion diffuse du côté du tissu cutané 1 par lequel le rayonnement optique a été introduit, et peut ainsi être détectée par l'un des capteurs optiques 9₁, 9₂.

Le capteur optique 9₁ mesure la partie rétrodiffusée 10₁ du rayonnement optique afin d'étudier la structure et/ou la composition de l'épiderme 2. Le capteur optique 9₂ mesure la partie rétrodiffusée 10₂ du rayonnement optique afin d'étudier la structure et/ou la composition du derme 3. Comme illustré sur la figure 1, la distance entre les capteurs 9₁, 9₂ et la source lumineuse 8 est choisie en fonction de la profondeur, dans le tissu cutané 1, de la zone d'étude considérée. De même, les propriétés du rayonnement optique, notamment sa longueur d'onde et son angle d'incidence sur le tissu cutané 1, sont choisies en fonction des constituants 4-6 du tissu cutané 1 à étudier.

La plupart des procédés et dispositifs actuels pour la spectroscopie par réflectance diffuse mettent uniquement en œuvre des rayonnements optiques d'intensité constante. Cependant, comme cela est développé dans l'article de Hasan Ayaz et al. [1], l'utilisation de rayonnements optiques modulés en intensité permettrait théoriquement d'obtenir davantage d'informations lors d'une spectroscopie par réflectance diffuse et de manière plus précise.

Toutefois, il n'est pas connu des inventeurs un dispositif et/ou un procédé existant dans l'état de l'art pour la spectroscopie par réflectance diffuse mettant en œuvre des rayonnements optiques modulés et présentant de meilleures performances que les procédés et dispositifs pour la spectroscopie par réflectance diffuse mettant uniquement en œuvre des rayonnements optiques non modulés au cours du temps.

Il existe donc un besoin pour un procédé et un dispositif présentant une bonne précision de la mesure de l' atténuation d'un rayonnement optique rétrodiffusé par un corps diffusant et/ou absorbant lors de sa spectroscopie par réflectance diffuse.

### Exposé de l'invention

L'invention concerne un procédé de mesure, par spectroscopie par réflectance diffuse, du coefficient d'atténuation d'une partie d'un corps diffusant et/ou absorbant, le procédé comprenant les étapes suivantes :
a) émission d'un rayonnement optique dont l'intensité et/ou la fréquence optique sont modulées, au moins une partie du rayonnement optique, dénommée signal de sonde, irradiant le corps,
b) réception d'une partie du signal de sonde diffusée et réfléchie par le corps, dénommée signal rétrodiffusé, et mesure de la longueur de parcours *d* du signal rétrodiffusé,
c) mesure de la réflectance *R* de la partie du corps traversée par le signal rétrodiffusé,
d) calcul du coefficient d'atténuation *µ* à partir de la longueur de parcours *d* et de la réflectance *R* mesurées.

L'invention met en œuvre un rayonnement optique modulé en intensité et/ou en fréquence optique pour une spectroscopie par réflectance diffuse afin de mesurer de façon précise la longueur de parcours *d* du signal rétrodiffusé. L'invention diffère donc des procédés de spectroscopie par réflectance diffuse connus de l'art antérieur qui ne sont pas adaptées à mesurer la longueur de parcours d'un signal rétrodiffusé. Dans les procédés de l'art antérieur connus des inventeurs, la longueur de parcours d'un signal rétrodiffusé est estimée par simulation numérique sans mesure d'un signal optique. Dans ces procédés connus de l'art antérieur, la mesure du coefficient d'atténuation pâtit de l'estimation approximative de la longueur de parcours du signal rétrodiffusé notamment lorsque le corps est diffusant.

Les inventeurs ont ainsi réussi à proposer un procédé mettant en œuvre une mesure par spectroscopie par réflectance diffuse au moyen d'un rayonnement modulé en intensité et/ou en fréquence optique qui présente des performances au moins aussi bonnes que celles des procédés connus qui mettent en œuvre des rayonnements optiques d'intensité constante. La modulation du rayonnement optique permet, sans complexifier la mise en œuvre du procédé, de mesurer la longueur de parcours *d.*

Avantageusement, le corps dont le coefficient d'atténuation est mesuré par le procédé selon l'invention est diffusant, homogène ou hétérogène. Enfin, la présente invention est simple à mettre en œuvre en pratique.

La «longueur de parcours *d* du signal rétrodiffusé » est la longueur moyenne des chemins optiques parcourus par le signal rétrodiffusé.

Le milieu diffusant peut être solide ou fluide, notamment liquide et/ou gazeux. Il peut comporter des éléments solides dispersés dans un fluide. Il peut comporter des gouttelettes d'un liquide en suspension dans un gaz.

Le milieu diffusant peut être un tissu biologique. Le tissu biologique peut notamment être une partie d'un corps animal, en particulier d'un corps humain, par exemple une peau. En variante, le tissu biologique peut être végétal.

De préférence, le rayonnement optique est laser.

La longueur d'onde du rayonnement optique est choisie en fonction du spectre d'absorption du ou des éléments contenus dans le corps dont l'observation par spectroscopie par réflectance diffuse est souhaitée. De préférence, le rayonnement optique présente une longueur d'onde comprise entre 400 nm et 1700 nm.

Le calcul du coefficient d'atténuation *µ* peut être réalisé à partir de la formule suivante, issue de la loi de Beer-Lambert : [Math 1] $R = \frac{{e}^{- μ d}}{{d}^{2}} .$

La formule [Math 1] est une approximation en première intention du coefficient d'atténuation *µ*. D'autres formules peuvent être utilisées, notamment provenant de modèle physique plus précis, par exemple prenant en considération le phénomène d'extinction.

Alternativement, le coefficient d'atténuation µ peut être décomposé en un coefficient d'absorption µₐ et un coefficient de diffusion µₛ, le calcul du coefficient d'atténuation µ pouvant être réalisé à partir de la formule suivante : [Math 2] $R = \frac{{z}_{0}}{4 π} . \left[\left({μ}_{eff}+\frac{1}{{r}_{1}}\right)\frac{{e}^{- {μ}_{eff} . {r}_{1}}}{{r}_{1}^{2}}+\left(1+\frac{4}{3}A\right)\left({μ}_{eff}+\frac{1}{{r}_{2}}\right)\frac{{e}^{- {μ}_{eff} . {r}_{2}}}{{{r}_{2}}^{2}}\right]$ avec ${z}_{0} = \frac{1}{{μ}_{a} + {μ}_{s} \left(1-g\right)}$ ${μ}_{eff} = \sqrt{3 {μ}_{a} \left({μ}_{a}+{μ}_{s}\left(1-g\right)\right)}$
où g est le facteur d'anisotropie, $A = \frac{1 + {r}_{i}}{1 - {r}_{i}}$ ${r}_{i} = - 1 , 440 {n}^{- 2} + 0 , 710 {n}^{- 1} + 0 , 0636 n + 0 , 668$
où *n* est égal au rapport de l'indice optique de la partie du corps traversée par le signal rétrodiffusé sur l'indice optique du milieu extérieur au corps,
*r₁* et *r₂* étant des facteurs dépendant de la longueur de parcours *d* et de la partie du corps traversée par le signal rétrodiffusé.

L'équation [Math 2] est déductible à partir de l'équation de la diffusion donnée par l'article de Farrell T. J., et al. : « A diffusion theory model of spatially resolved, steady-state diffuse reflectance for the noninvasive determination of tissue optical properties in vivo », Med Phys., 1992, 19(4), 879-88.

Il est également envisageable d'utiliser un modèle de type Monte-Carlo pour le calcul du coefficient d'atténuation *µ*, notamment dans le cas où l'approximation de la diffusion n'est plus valide, c'est-à-dire que le coefficient d'absorption *µₐ* est négligeable par rapport au coefficient de diffusion réduit *µₛ*' et que la distance L entre le point où pénètre dans le corps le signal de sonde et le point où émerge du corps le signal rétrodiffusé est très petite devant l'inverse du coefficient de diffusion réduit *µₛ*'. Le coefficient de diffusion réduit *µₛ'* étant égal à µ*ₛ*(1 - *g*)*,* où *g* est le facteur d'anisotropie. L'utilisation d'un modèle de type Monte Carlo permet de simuler la trajectoire des photons en faisant intervenir des procédés aléatoires. Chaque photon est considéré comme une particule individuelle dont on suit le trajet et les interactions. Un résultat statistique est ensuite obtenu en envoyant un très grand nombre de photons, typiquement de l'ordre de 10⁶ photons.

La mesure de la réflectance *R* peut comprendre la mesure de l'intensité moyenne *iᵣ* du signal rétrodiffusé et le calcul de la réflectance *R* par la formule suivante : [Math 3] $R = \frac{{i}_{r}}{{i}_{e}}$ où *iₑ* est l'intensité moyenne du signal de sonde irradiant le corps.

L'intensité moyenne d'un signal optique est la valeur moyenne de l'intensité dudit signal optique intégrée sur une période de modulation dudit signal optique.

En variante, la mesure de la réflectance *R* comprend l'émission d'un signal de sonde supplémentaire de même longueur d'onde que le rayonnement optique et irradiant le corps, suivie de la mesure de l'intensité moyenne *iᵣ'* d'une partie du signal de sonde supplémentaire diffusée et réfléchie par le corps, dénommée signal rétrodiffusé supplémentaire, puis du calcul de la réflectance *R* par la formule suivante : [Math 4] $R = \frac{{i}_{r} ′}{{i}_{e} ′}$ où *iₑ'* est l'intensité moyenne du signal de sonde supplémentaire irradiant le corps.

De préférence, le signal de sonde supplémentaire est d'intensité constante.

Selon un autre variante, l'étape a) comprend la modulation de la fréquence optique du rayonnement optique selon une rampe de hauteur *h* et de durée *t,* et comprend la séparation du rayonnement optique en le signal de sonde et en un oscillateur local, l'oscillateur local n'irradiant pas le corps,
la mesure de la réflectance *R* comprenant le mélange du signal rétrodiffusé et de l'oscillateur local pour former un battement électromagnétique suivi du calcul de la réflectance *R* à partir de l'amplitude de modulation de l'intensité du battement électromagnétique et/ou à partir de la valeur de la composante continue de l'intensité du battement électromagnétique. La réflectance *R* peut être obtenue à partir de l'amplitude de la transformée de Fourier de l'intensité du battement électromagnétique.

De préférence, l'intensité du rayonnement optique est modulée sinusoïdalement.

De préférence, la fréquence de modulation de l'intensité du rayonnement optique est comprise entre 10 et 1000 MHz. Avantageusement, plus la fréquence de modulation est élevée, meilleure est la précision de la mesure.

De préférence, la mesure de l'intensité moyenne *iᵣ* du signal rétrodiffusé ou de l'intensité moyenne *iᵣ'* du signal rétrodiffusé supplémentaire est réalisée par un démodulateur photonique assisté par courant ou par une photodiode à jonction PIN.

Selon un premier mode de mise en œuvre, l'intensité du rayonnement optique peut être modulée sinusoïdalement à une fréquence de modulation *f_{mod},* la mesure de la longueur de parcours *d* peut comprendre la mesure du déphasage *α* entre le signal rétrodiffusé et le rayonnement optique émis, suivie du calcul de la longueur de parcours d à partir de la formule suivante : [Math 5] $d = c . \frac{α}{4 . π . {f}_{mod}}$ où c est la vitesse de la lumière dans le vide.

De préférence, la mesure du déphasage *α* est réalisée par un démodulateur photonique assisté par courant.

Selon un deuxième mode de mise en œuvre, pour mesurer la longueur de parcours *d,* l'étape a) comprend la modulation de la fréquence optique du rayonnement optique selon une rampe de hauteur *h* et de durée *t,* et,
l'étape a) comprend la séparation du rayonnement optique en le signal de sonde et en un oscillateur local, l'oscillateur local n'irradiant pas le corps,
l'étape b) comprend le mélange du signal rétrodiffusé et de l'oscillateur local pour former un battement électromagnétique suivi du calcul de la longueur de parcours *d* à partir de la formule suivante : [Math 6] ${f}_{bat} = h . \frac{d}{c . t}$
où *f_{bat}* est la fréquence moyenne du battement électromagnétique et *c* est la vitesse de la lumière dans le vide.

De préférence, la hauteur *h* est comprise entre 12 et 40 GHz.

De préférence, la durée *t* est comprise entre 0,1 et 10 ms.

De préférence, la séparation du rayonnement optique est réalisée de sorte que l'intensité de l'oscillateur local lors du mélange formant le battement électromagnétique est sensiblement égale à l'intensité du signal rétrodiffusé lors dudit mélange. Avantageusement, la sensibilité de la spectroscopie par réflectance diffuse est alors maximisée.

L'obtention de la fréquence *f_{bat}* du battement électronique peut comprendre la mesure de l'intensité du battement électromagnétique au cours du temps. De préférence, la mesure de l'intensité du battement électromagnétique est réalisée par une photodiode à jonction PIN, de préférence deux photodiodes à jonction PIN connectées électriquement en séries. La ou les photodiodes à jonction PIN peuvent être formée de germanium, de silicium ou d'arséniure d'indium-gallium. Le silicium est privilégié pour un rayonnement optique de longueur d'onde comprise entre 400 et 940 nm. L'arséniure d'indium-gallium est privilégié pour un rayonnement optique de longueur d'onde comprise entre 1 et 2,5 µm.

De préférence, la fréquence *f_{bat}* du battement électromagnétique est obtenue par un traitement comportant une transformée de Fourier de l'intensité du battement électromagnétique.

La présente invention concerne également un dispositif pour réaliser le procédé selon l'invention selon le premier mode de mise en œuvre, le dispositif comprenant :
- une source d'émission lumineuse configurée pour émettre un rayonnement optique, de préférence ayant une longueur d'onde comprise entre 400 nm et 1700 nm, modulé sinusoïdalement en intensité et dont au moins une partie, dénommée signal de sonde, est destinée à irradier un corps diffusant et/ou absorbant,
- un détecteur optique configuré pour mesurer le déphasage *α* entre une partie du signal de sonde diffusée et réfléchie par le corps, dénommée signal rétrodiffusé, et le rayonnement optique émis, et pour mesurer l'intensité moyenne du signal rétrodiffusé, la source d'émission lumineuse et le détecteur optique étant agencés pour être disposés d'un même côté du corps,
- une unité de traitement de données configurée pour calculer la longueur de parcours *d* à partir de la formule [Math 5], et pour calculer le coefficient d'atténuation *µ* à partir de la longueur de parcours *d* calculée et de la réflectance *R* de la partie du corps traversée par le signal rétrodiffusé mesurée à partir de l'intensité moyenne mesurée par le détecteur optique.

De préférence, le détecteur optique comprend au moins un démodulateur photonique assisté par courant, de préférence une pluralité de démodulateurs photoniques assistés par courant. De préférence, les démodulateurs photoniques assistés par courant sont agencés périodiquement dans un plan.

Avantageusement, un démodulateur photonique assisté par courant est apte à mesurer un déphasage entre le signal rétrodiffusé et le rayonnement optique émis en ne recevant que le signal rétrodiffusé. Pour cela, le démodulateur photonique assisté par courant peut être calibré avec une fréquence de détection multiple de la fréquence de modulation en intensité du rayonnement optique. Il est aussi apte à mesurer l'intensité d'un signal optique au cours du temps. Ainsi, si ledit signal optique est d'intensité constante alors l'intensité mesurée est égale à son intensité moyenne. Autrement, si ledit signal optique est modulé en intensité alors l'intensité moyenne mesurée par le démodulateur photonique peut être déterminée par intégration de l'intensité mesurée durant une période complète de modulation.

De préférence, le détecteur optique comprend au moins une photodiode à jonction PIN, de préférence une pluralité de photodiodes à jonction PIN. De préférence, les photodiodes à jonction PIN sont agencées périodiquement dans un plan. La ou les photodiodes à jonction PIN peuvent être formée de germanium, de silicium ou d'arséniure d'indium-gallium.

De préférence, la distance entre la ou au moins une, de préférence chacune, des photodiodes à jonction PIN et le démodulateur photonique assisté par courant le plus proche est inférieure à 100 µm, de préférence à 10 µm. Ainsi, les photons collectés par ledit démodulateur photonique assisté par courant et par ladite photodiode à jonction PIN présentent, sensiblement, la même longueur de parcours et ont traversé la même partie du corps diffusant et/ou absorbant.

De préférence, les démodulateurs photoniques sont alternés avec les photodiodes à jonction PIN. Par exemple, les démodulateurs photoniques peuvent être alternés en quinconce avec les photodiodes à jonction PIN. Notamment, les démodulateurs photoniques et les photodiodes à jonction peuvent être disposées à la manière d'un damier. Les démodulateurs photoniques peuvent également être alternés avec les photodiodes à jonction PIN de sorte que chaque démodulateur photonique est entouré de huit photodiodes à jonction PIN adjacentes.

Une pluralité de démodulateurs photoniques assistés par courant et/ou une pluralité de photodiodes à jonction PIN permet, avantageusement, de collecter plusieurs signaux optiques rétrodiffusés provenant d'un même signal de sonde et ayant chacun traversés des parties différentes du corps diffusant et/ou absorbant. Ainsi, cela permet de mesurer facilement et rapidement la longueur de parcours et le coefficient d'atténuation pour différentes parties d'un même corps diffusant et/ou absorbant, ce qui est particulièrement intéressant lorsque le corps est hétérogène et/ou stratifié.

L'invention concerne également un dispositif pour réaliser le procédé selon l'invention selon le deuxième mode de mise en œuvre, le dispositif comprenant :
- une source d'émission lumineuse configurée pour émettre un rayonnement optique cohérent, de préférence ayant une longueur d'onde comprise entre 400 nm et 1700 nm, dont la fréquence optique est modulée selon une rampe de hauteur *h* et de durée *t,*
- un séparateur de faisceau configuré pour séparer le rayonnement optique en un signal de sonde destiné à irradier un corps diffusant et/ou absorbant et au moins un oscillateur local,
- une unité de détection optique comportant au moins un multiplexeur pour mélanger l'oscillateur local avec une partie du signal de sonde diffusée et réfléchie par le corps, dénommée signal rétrodiffusé, afin de former un battement électromagnétique, l'unité de détection optique comportant en outre au moins un récepteur optique configuré pour mesurer l'intensité au cours du temps du battement électromagnétique et pour mesurer l'intensité moyenne du signal rétrodiffusé, la source d'émission lumineuse, le séparateur de faisceau et l'unité de détection optique étant agencés pour être disposés d'un même côté du corps,
- une unité de traitement de données configurée pour déterminer la fréquence moyenne *f_{bat}* du battement électromagnétique à partir de l'intensité mesurée dudit battement électromagnétique, pour calculer la longueur de parcours *d* à partir de la formule [Math 6], et calculer le coefficient d'atténuation µ à partir de la longueur de parcours *d* calculée et de la réflectance *R* de la partie du corps traversée par le signal rétrodiffusé mesurée à partir de l'intensité moyenne du signal rétrodiffusé mesurée par le récepteur optique ou à partir de l'intensité du battement électromagnétique.

De préférence, le séparateur de faisceau est configuré pour que la phase et la fréquence fondamentale de l'oscillateur local et la phase et la fréquence fondamentale du signal de sonde soient égales respectivement.

De préférence, l'unité de détection optique étant configurée pour mesurer séparément la composante continue de l'intensité du battement électromagnétique et la composante alternative de l'intensité du battement électromagnétique. Ainsi, la réflectance *R* peut être calculée à partir de l'amplitude de modulation de la composante alternative et/ou à partir de la valeur de la composante continue. Cette séparation simplifie également la détermination de la fréquence moyenne f_{bat}.

De préférence, l'unité de détection optique comprend une pluralité de récepteurs optiques et autant de multiplexeur que de récepteurs optiques, le séparateur de faisceau est configuré pour séparer le rayonnement optique en un signal de sonde et autant d'oscillateurs locaux que de récepteurs optiques, chacun des multiplexeurs est configuré pour mélanger un des oscillateurs locaux avec un signal rétrodiffusé, afin de former un battement électromagnétique dirigé vers un des récepteurs optiques. Une pluralité de récepteurs optiques permet, avantageusement, de collecter plusieurs signaux optiques rétrodiffusés provenant d'un même signal de sonde et ayant chacun traversé une partie différente du corps diffusant et/ou absorbant. Ainsi, cela permet de mesurer facilement et rapidement la longueur de parcours et le coefficient d'atténuation pour différentes parties d'un même corps diffusant et/ou absorbant, ce qui est particulièrement intéressant lorsque le corps est hétérogène et/ou stratifié.

De préférence, le dispositif comprend au moins un guide d'onde pour conduire l'oscillateur local du séparateur de faisceau jusqu'à l'unité de détection optique. Le guide d'onde peut être en un matériau choisi parmi le silicium, le nitrure de silicium, le dioxyde de silicium et leurs mélanges. Le guide d'onde peut être une fibre optique.

De préférence, le récepteur optique comprend au moins une photodiode à jonction PIN, de préférence deux photodiodes à jonction PIN connectées électriquement en séries. La ou les photodiodes à jonction PIN peuvent être formée de germanium, de silicium ou d'arséniure d'indium-gallium. De préférence, le récepteur optique comprend une amplificateur optique pour amplifier la différence de courant électrique entre les deux photodiodes à jonction PIN.

De préférence, l'unité de traitement de données est configurée de sorte que le calcul du coefficient d'atténuation µ est réalisé à partir de la formule [Math 1] ou [Math 2].

De préférence, la source d'émission lumineuse est configurée pour émettre un signal de sonde supplémentaire de même longueur d'onde que le rayonnement optique, d'intensité constante et destiné à irradier le corps.

D'autres avantages et caractéristiques ressortiront mieux à la lecture de la description détaillée, faite à titre illustratif et non limitatif, et en référence aux figures suivantes.

### Brève description des dessins

[Fig 1] La figure 1 est une représentation schématique en coupe transversale d'un exemple de mise en œuvre d'une mesure par spectroscopie par réflectance diffuse connue de l'art antérieur.
[Fig 2] La figure 2 est une représentation schématique en coupe transversale d'un exemple de mesure par spectroscopie par réflectance diffuse selon le premier mode de mise en œuvre du procédé selon l'invention.
[Fig 3] La figure 3 est un graphique représentant l'évolution au cours du temps de l'intensité lumineuse du signal de sonde et du signal rétrodiffusé lors de la mise en œuvre du procédé illustré sur la figure 2.
[Fig 4] La figure 4 est une représentation schématique en coupe transversale d'un exemple de mesure par spectroscopie par réflectance diffuse selon le deuxième mode de mise en œuvre du procédé selon l'invention.
[Fig 5] La figure 5 est un graphique représentant l'évolution de l'intensité lumineuse du signal de sonde supplémentaire et du signal rétrodiffusé supplémentaire au cours du temps.
[Fig 6] La figure 6 est une représentation schématique en vue du dessus d'un détecteur optique d'un dispositif selon l'invention, le détecteur optique comprenant une pluralité de démodulateurs photoniques assistés par courant et une pluralité de photodiodes à jonction PIN.
[Fig 7] La figure 7 est une représentation schématique en coupe transversale d'un exemple de dispositif comprenant le détecteur optique selon la figure 6 pour une mesure par spectroscopie par réflectance diffuse.
[Fig 8] La figure 8 est une représentation schématique en vue du dessus d'un détecteur optique d'un dispositif selon l'invention, le détecteur optique comprenant une pluralité de démodulateurs photoniques assistés par courant.
[Fig 9] La figure 9 est une représentation schématique en coupe transversale d'un troisième exemple de mesure par spectroscopie par réflectance diffuse selon l'invention.
[Fig 10] La figure 10 est un graphique représentant l'évolution selon une rampe de la fréquence optique d'un signal de sonde.

### Description détaillée

Dans les figures, les différents éléments constituant le dispositif selon l'invention ainsi que le milieu diffusant et/ou absorbant n'ont pas nécessairement été représentés à l'échelle, par souci de clarté du dessin.

La figure 1 a été décrite ci-dessus.

On a illustré à la figure 2 un exemple de réalisation d'un dispositif 11 pour la spectroscopie par réflectance diffuse d'un corps 1. Le dispositif 11 comprend une source d'émission lumineuse 15 et un détecteur optique 20 tous deux en contact surfacique avec le corps 1 diffusant et/ou absorbant. Le dispositif 11 comprend également une unité de traitement de données 25 connectée à la source d'émission lumineuse 15 et au détecteur optique 20.

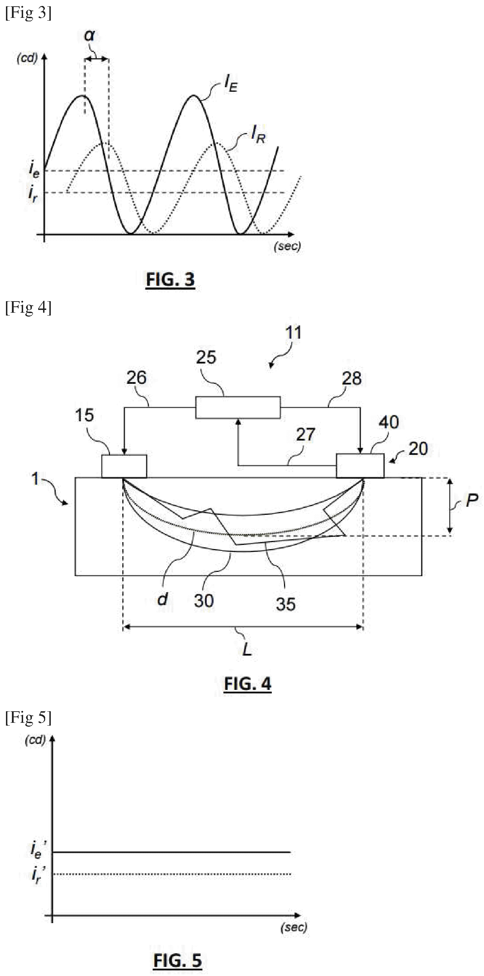

Sous instructions 26 de l'unité de traitement de données 25, la source d'émission lumineuse 15 émet un rayonnement optique de longueur d'onde comprise entre 400 nm et 1700 et modulé en intensité. Au moins une partie, de préférence l'entièreté, du rayonnement optique est dirigée pour irradier le corps 1. Cette partie est dénommée signal de sonde. L'évolution de l'intensité du signal de sonde en fonction du temps est représentée par la figure 3. Le signal de sonde comprend une composante continue d'intensité constante égale *à i_{DC}* et une composante alternative, modulée sinusoïdalement en intensité, avec une amplitude égale à *i_{AC}* et une fréquence égale à *f_{mod}.* Aussi, l'intensité moyenne au cours du temps *iₑ* du signal de sonde est égale *à i_{DC}.* L'évolution de l'intensité *I_{E}* du signal de sonde au cours du temps est donnée par la formule suivante : [Math 7] ${I}_{E} \left(t\right) = {i}_{DC} + {i}_{AC} . sin \left(2{πf}_{mod}t\right) .$

Au cours de son trajet dans le corps 1, le signal de sonde est en partie diffusé et réfléchi par le corps 1. Le détecteur optique 20 reçoit et collecte une partie du signal de sonde diffusée et réfléchie par le corps 1, dénommée signal rétrodiffusé. L'évolution de l'intensité du signal rétrodiffusé en fonction du temps est représentée par la figure 3. Le signal rétrodiffusé comprend une composante continue d'intensité constante égale *à R.i_{DC}* et une composante alternative, modulée sinusoïdalement en intensité, avec une amplitude égale à *R.i_{AC}* et une fréquence égale à *f_{mod},* où *R* est la réflectance de la partie du corps 1 traversée par le signal rétrodiffusé. Le signal rétrodiffusé présente une différence de phase *α* avec le rayonnement optique émis par la source d'émission lumineuse 15. L'intensité moyenne au cours du temps *iᵣ* du signal rétrodiffusé est égale *à R.i_{DC}.* L'évolution de l'intensité *I_{R}* du signal rétrodiffusé au cours du temps est donnée par la formule suivante : [Math 8] ${I}_{R} \left(t\right) = R . {i}_{DC} + R . {i}_{AC} . sin \left(2{πf}_{mod}t+α\right) .$

On a illustré à la figure 2 l'enveloppe 30 du signal rétrodiffusé. L'enveloppe 30 contient l'ensemble statistique des trajectoires 35 des photons reçus et collectés par le détecteur optique 20. Le signal rétrodiffusé présente une longueur de parcours *d,* correspondant à la longueur moyenne des trajectoires 35 des photons reçus et collectés par le détecteur optique 20. Le signal rétrodiffusé a également une profondeur *P* correspondant à la profondeur moyenne atteinte par les photons reçus et collectés par le détecteur optique 20. Cette profondeur *P* dépend de la distance *L* entre le point où pénètre dans le corps 1 le signal de sonde et le point où émerge du corps le signal rétrodiffusé, plus cette distance *L* augmente, plus la profondeur *P* augmente.

Dans le mode de réalisation illustré à la figure 2, le détecteur optique 20 comprend un démodulateur photonique assisté par courant 40, également dénommé « Current-Assisted Photonic Demodulator » (CAPD) en anglais, et une photodiode à jonction PIN 45. Le démodulateur photonique assisté par courant 40 est accolé à la photodiode à jonction PIN 45. Le démodulateur photonique assisté par courant 40 est configuré pour mesurer le déphasage *α* entre le signal rétrodiffusé et le rayonnement optique émis par la source d'émission lumineuse 15. La photodiode à jonction PIN 45 est configurée pour mesurer l'intensité moyenne au cours du temps *iᵣ* du signal rétrodiffusé. Une fois le déphasage *α* et l'intensité moyenne *iᵣ* mesurés, le détecteur optique 20 les transmet à l'unité de traitement 25 de données via la liaison 27. Si besoin, l'unité de traitement de données 25 peut commander le détecteur optique 25 via la liaison 28, par exemple pour activer/désactiver le détecteur optique 25 et/ou pour calibrer le démodulateur photonique assisté par courant 40, notamment sa fréquence de détection.

Après réception du déphasage *α* et de l'intensité moyenne *iᵣ* mesurés, l'unité de traitement de données 25 calcule la longueur de parcours *d* à partir de la formule [Math 5] donnée précédemment et calcule la réflectance *R* égale au rapport de l'intensité moyenne *iᵣ* du signal rétrodiffusé sur l'intensité moyenne *iₑ* du signal de sonde. L'unité de traitement de données 25 calcule ensuite le coefficient d'atténuation *µ* à partir de la formule [Math 1] de Beer-Lambert ou de la formule [Math 2].

On a illustré sur la figure 4 un autre mode de réalisation d'un dispositif 11 selon l'invention pour la spectroscopie par réflectance diffuse d'un corps 1. Selon ce mode de réalisation, le détecteur optique 20 peut être exempt de photodiode à jonction PIN 45. Similairement au mode de réalisation illustré à la figure 2, le démodulateur photonique assisté par courant 40 mesure le déphasage *α* entre le signal rétrodiffusé et le rayonnement optique émis par la source d'émission lumineuse 15 et l'unité de traitement de données 25 calcule la longueur de parcours d du signal rétrodiffusé à partir de la formule [Math 5] donnée précédemment.

Le procédé mettant en œuvre le dispositif 11 illustré sur la figure 4 diffère de celui illustré sur la figure 2 en la mesure de la réflectance *R* de la partie du corps 1 traversée par le signal rétrodiffusé. Pour cette mesure, la source d'émission lumineuse 15 émet vers le corps 1, un signal de sonde supplémentaire de même longueur d'onde que le rayonnement optique. Le signal de sonde supplémentaire est d'intensité constante *iₑ'* au cours du temps, comme cela est représentée par la figure 5. Pour cette mesure de la réflectance *R,* les positionnements de la source d'émission lumineuse 15 et du détecteur optique 20 sont les mêmes que pour l'émission du rayonnement optique et la réception du signal de sonde.

Au cours de son trajet dans le corps 1, le signal de sonde supplémentaire est en partie diffusé et réfléchi par le corps 1. Le détecteur optique 20 reçoit et collecte une partie du signal de sonde supplémentaire diffusée et réfléchie par le corps 1, dénommée signal rétrodiffusé supplémentaire. Le signal rétrodiffusé supplémentaire est d'intensité constante *iᵣ'* égale à *R.iₑ'* au cours du temps, où *R* est la réflectance de la partie du corps 1 traversée par le signal rétrodiffusé supplémentaire. Les trajectoires 35 des photons du signal rétrodiffusé supplémentaire reçus et collectés par le détecteur optique 20 sont similaires aux trajectoires 35 des photons du signal rétrodiffusé. Aussi, le signal rétrodiffusé supplémentaire a la même enveloppe 30, la même longueur de parcours *d* et la même profondeur *P* que le signal rétrodiffusé.

Dans le mode de réalisation illustré à la figure 4, le démodulateur photonique assisté par courant 40 mesure l'intensité *iᵣ'* du signal rétrodiffusé supplémentaire. Ensuite, l'unité de traitement de données 25 calcule la réflectance *R* égale au rapport de l'intensité *iᵣ'* du signal rétrodiffusé supplémentaire sur l'intensité *iₑ'* du signal de sonde supplémentaire. L'unité de traitement de données 25 calcule ensuite le coefficient d'atténuation µ à partir, par exemple, de la formule [Math 1] issue de la loi de Beer-Lambert ou de la formule [Math 2].

La mesure de la réflectance *R* par l'émission d'un signal de sonde supplémentaire peut être effectuée avant ou après la mesure du déphasage *α* entre le signal rétrodiffusé et le rayonnement optique, car elle est indépendante de cette dernière.

Dans les modes de réalisation présentés sur les figures 2 et 4, le détecteur optique 20 ne comprend qu'un unique démodulateur photonique assisté par courant 40, et le cas échéant, une unique photodiode à jonction PIN 45. Aussi, ces modes de réalisation ne sont pas adaptés à mesurer simultanément le coefficient d'atténuation *µ* de différentes parties du corps 1. Ils sont adaptés à ne mesurer le coefficient que d'une unique partie du corps 1, ladite partie correspondant à l'enveloppe 30 du signal rétrodiffusé collecté.

Selon d'autres modes de réalisation, le détecteur optique 20 peut comprendre plusieurs démodulateurs photoniques assistés par courant 40, et le cas échéant, plusieurs photodiodes à jonction PIN 45.

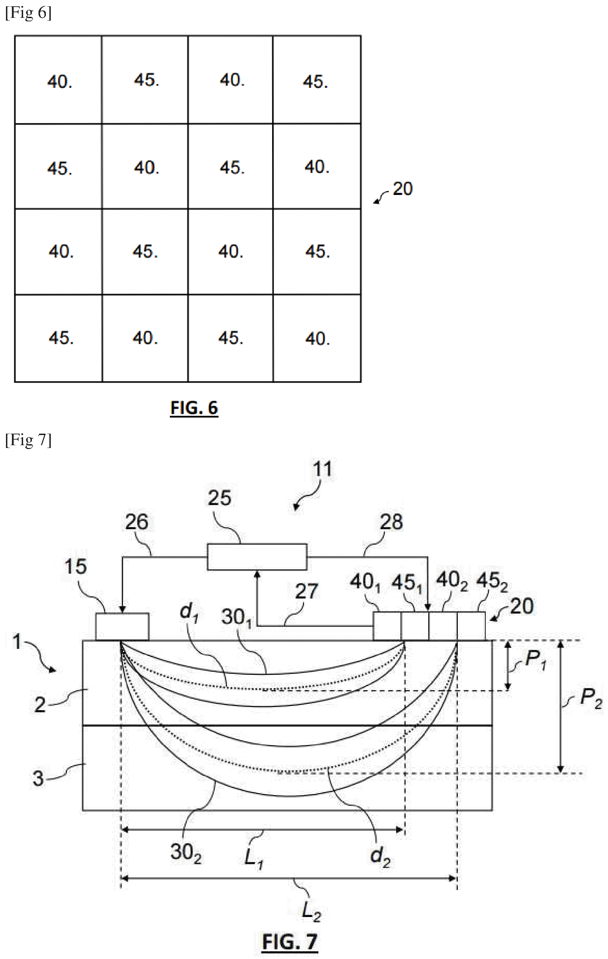

Par exemple, comme cela est illustré sur la figure 6, le détecteur optique 20 peut comprendre une pluralité de démodulateurs photoniques assistés par courant 40 et une pluralité de photodiodes à jonction PIN 45. Les démodulateurs photoniques assistés par courant 40 sont accolés en quinconce avec les photodiodes à jonction PIN 45 dans un plan. Ce détecteur optique 20 est adapté pour mesurer simultanément le coefficient d'atténuation *µ* de différentes parties du corps 1.

On a illustré à la figure 7 un mode d'utilisation d'un dispositif 11 selon l'invention comprenant le détecteur optique 20 illustré à la figure 6. Similairement au mode illustré par la figure 2, la source d'émission lumineuse 15 émet un rayonnement optique dont une partie, dite signal de sonde, illumine le corps 1. Une première partie du signal de sonde diffusée et réfléchie par le corps 1, dite premier signal rétrodiffusé, est reçue par un premier démodulateur photonique assisté par courant 40₁ et une première photodiode à jonction PIN 45₁. Une deuxième partie du signal de sonde diffusée et réfléchie par le corps 1, dite deuxième signal rétrodiffusé, est reçue par un deuxième démodulateur photonique assisté par courant 40₂ et une deuxième photodiode à jonction PIN 45₂.

Le premier signal rétrodiffusé présente une enveloppe 30₁ différente de l'enveloppe 30₂ du deuxième signal rétrodiffusé. Notamment, le premier signal rétrodiffusé a une profondeur *P₁* inférieure à la profondeur *P₂* du deuxième signal rétrodiffusé. Cela est dû aux distances *L₁,* respectivement *L₂*, entre le point où le signal de sonde pénètre dans le corps 1 et le point où le premier signal rétrodiffusé, respectivement le deuxième signal rétrodiffusé, émerge du corps 1. Autrement dit, les enveloppes 30₁ et 30₂ sont fonction du positionnement des démodulateurs photoniques assistés par courant 40₁ et 40₂ et des photodiodes à jonction PIN 45₁ et 45₂ relativement à la source d'émission lumineuse 15. Ainsi, la partie du corps 1, dite première partie, traversée par le premier signal rétrodiffusé diffère de la partie du corps 1, dite deuxième partie, traversée par le deuxième signal rétrodiffusé.

La longueur de parcours *d₁* du premier signal rétrodiffusé et le coefficient d'atténuation *µ₁* de la première partie sont obtenus similairement à l'exemple illustré à la figure 2. Il en est de même la longueur de parcours *d₂* du deuxième signal rétrodiffusé et le coefficient d'atténuation *µ₂* de la deuxième partie.

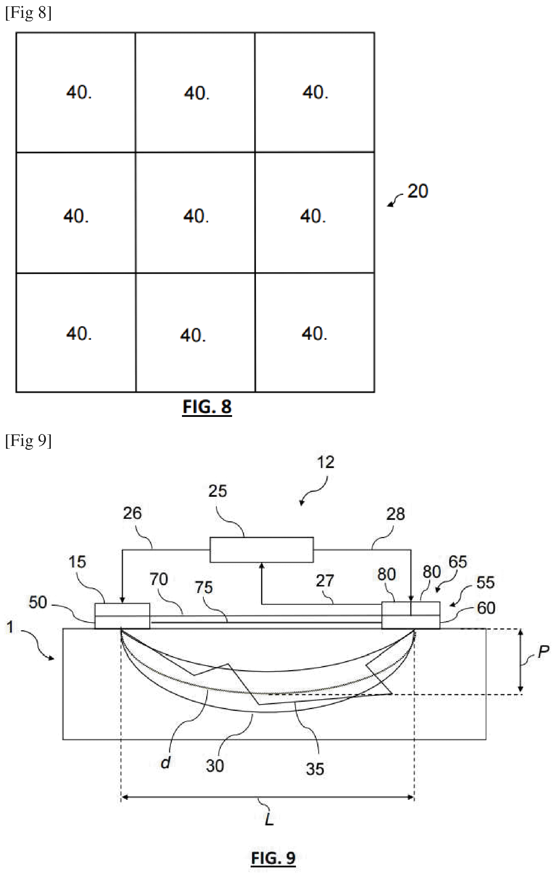

Selon un autre exemple illustré à la figure 8, le détecteur optique 20 peut comprendre une pluralité de démodulateurs photoniques assistés par courant 40 accolés les uns aux autres dans un plan. Chaque démodulateur photonique assisté par courant 40 est configuré pour recevoir un signal rétrodiffusé similairement au mode de réalisation illustré à la figure 4, chacun desdits signaux rétrodiffusé ayant traversé une partie du corps 1 distincte des parties traversées par les autres signaux rétrodiffusés. Ainsi, le détecteur optique 20 permet la mesure d'un coefficient d'atténuation *µ* pour chacune des partie du corps 1 traversées par les signaux rétrodiffusés reçus par les démodulateurs photoniques assistés par courant 40.

On a illustré à la figure 9 un exemple de dispositif 12 selon l'invention pour la spectroscopie par réflectance diffuse d'un corps 1. Le dispositif 12 comprend une source d'émission lumineuse 15, un séparateur de faisceau 50 et une unité de détection optique 55 tous agencés du même côté du corps 1 diffusant et/ou absorbant. Le dispositif 12 comprend également une unité de traitement de données 25 connectée à la source d'émission lumineuse 15 et à l'unité de détection optique 55.

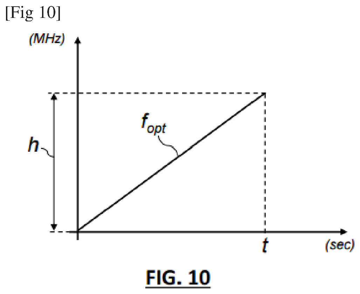

Sous instructions 26 de l'unité de traitement de données 25, la source d'émission lumineuse 15 émet un rayonnement optique de longueur d'onde comprise entre 400 nm et 1700 nm et dont la fréquence optique est modulée. On a illustré à la figure 10 l'évolution de la fréquence optique *fₒₚₜ* au cours du temps qui suit une rampe de hauteur égale à *h* et de durée *t.*

Le rayonnement optique est séparé par le séparateur de faisceau 50 en un signal de sonde qui irradie le corps 1 et en un oscillateur local 75 qui est dirigé par un guide d'onde 70 jusqu'à l'unité de détection optique 55. L'évolution de l'intensité du signal de sonde en fonction du temps est similaire à celle représentée à la figure 3. Le signal de sonde comprend une composante continue d'intensité constante égale *à i_{DC}* et une composante alternative, modulée sinusoïdalement en intensité, avec une amplitude égale à *i_{AC}* et une fréquence optique *fₒₚₜ* également modulée. Aussi, l'intensité moyenne au cours du temps *iₑ* du signal de sonde est égale à *i_{DC}.* L'évolution de l'intensité *I_{E}* du signal de sonde au cours du temps est similaire à la formule [Math 6]. L'intensité de l'oscillateur local 75 évolue similairement à l'intensité I_{E} du signal de sonde et ne diffère qu'en amplitude.

Le signal de sonde pénètre dans le corps 1, puis est en partie diffusé et réfléchi par le corps 1. L'unité de détection optique 55 reçoit une partie du signal de sonde diffusée et réfléchie par le corps 1, dénommée signal rétrodiffusé. Le signal rétrodiffusé comprend une composante continue d'intensité constante égale *à R.i_{DC}* et une composante alternative, modulée sinusoïdalement en intensité, avec une amplitude égale à *R.i_{AC}* et une fréquence optique *fₒₚₜ également* modulée, où *R* est la réflectance de la partie du corps 1 traversée par le signal rétrodiffusé. Le signal rétrodiffusé présente une différence de phase de *α* avec l'oscillateur local 75. Le déphasage *α* est caractéristique de la durée supplémentaire induite par le corps 1 sur le signal rétrodiffusé pour atteindre l'unité de détection optique 55. L'intensité moyenne au cours du temps *iᵣ* du signal rétrodiffusé est égale à *R. i_{DC}.* L'évolution de l'intensité *I_{R}* du signal rétrodiffusé au cours du temps est donnée par la formule suivante [Math 7].

L'unité de détection optique 55 comporte un multiplexeur 60 qui mélange l'oscillateur local 75 avec le signal rétrodiffusé. L'oscillateur local 75 et le signal rétrodiffusé étant déphasés, leur mélange forme, par interférence, un battement électromagnétique.

L'unité de détection optique 55 comprend également un récepteur optique 65 comprenant deux photodiodes à jonction PIN 80 en série. Les photodiodes à jonction PIN 80 reçoivent le battement électromagnétique et mesurent l'évolution de son intensité au cours du temps. Le récepteur optique 65 transmet l'évolution de l'intensité mesurée à l'unité de traitement de données 25.

L'unité de traitement de données 25 détermine la fréquence moyenne *f_{bat}* du battement électromagnétique à partir de la transformée de Fourier de son intensité mesurée. Comme cela est expliqué dans la demande de brevet FR 2113799 A, la fréquence moyenne *f_{bat}* du battement électromagnétique est caractéristique, notamment proportionnelle, de la durée moyenne du trajet des photons du signal de sonde pour traverser le corps 1. La fréquence moyenne *f_{bat}* est donc également caractéristique de la longueur de parcours *d* du signal rétrodiffusé. Ainsi, l'unité de traitement de données 25 calcule la longueur de parcours *d* à partir de la fréquence moyenne *f_{bat}* et de la formule [Math 5] donnée précédemment.

Le dispositif 12 mesure également la réflectance *R* de la partie du corps 1 traversée par le signal rétrodiffusé. Pour cette mesure, les positionnements de la source d'émission lumineuse 15 et de l'unité de détection optique 55 sont les mêmes que pour l'émission du rayonnement optique et la réception du signal de sonde. Cette mesure comprend l'émission par la source d'émission lumineuse 15 d'un signal de sonde supplémentaire irradiant le corps 1. Préférentiellement, le signal de sonde supplémentaire est d'intensité constante.

Au cours de son trajet dans le corps 1, le signal de sonde supplémentaire est en partie diffusé et réfléchi par le corps 1. L'unité de détection optique 55 reçoit et collecte une partie du signal de sonde supplémentaire diffusée et réfléchie par le corps 1, dénommée signal rétrodiffusé supplémentaire. Le signal rétrodiffusé supplémentaire est d'intensité proportionnelle à l'intensité du signal de sonde supplémentaire émis selon un facteur égal à la réflectance *R.* Comme expliqué précédemment, le signal rétrodiffusé supplémentaire a la même enveloppe 30, la même longueur de parcours *d* et la même profondeur *P* que le signal rétrodiffusé.

Les photodiodes à jonction PIN 80 mesurent l'intensité du signal rétrodiffusé supplémentaire. L'unité de détection optique 55 transmet l'intensité mesurée à l'unité de traitement de données 25 qui calcule alors la réflectance *R* égale au rapport de l'intensité du signal rétrodiffusé supplémentaire sur l'intensité du signal de sonde supplémentaire. L'unité de traitement de données 25 calcule ensuite le coefficient d'atténuation *µ* à partir de la formule [Math 1] de Beer-Lambert ou de la formule [Math 2].

La mesure de la réflectance *R* par l'émission d'un signal de sonde supplémentaire peut être effectuée avant ou après la mesure de la fréquence moyenne *f_{bat}* du battement électromagnétique, car elle est indépendante de cette dernière.

En variante, la réflectance *R* peut être mesurée à partir de l'amplitude de modulation de l'intensité du battement électromagnétique et/ou à partir de la valeur de la composante continue de l'intensité du battement électromagnétique. Notamment, l'évolution de l'intensité *I_{bat}* du battement électromagnétique au cours du temps est donnée par la formule suivante : [Math 8] ${I}_{bat} \left(t\right) = {i}_{tot} + \left(R-1\right) . {i}_{DC} + \sqrt{R . {i}_{DC} . \left({i}_{tot}-{i}_{DC}\right)} . sin \left(2{πf}_{bat}t+ϕ\right) ,$ où *ϕ est une phase quelconque, iₜₒₜ* est l'intensité moyenne du rayonnement optique et *i_{DC}* est l'intensité moyenne du signal de sonde.

Comme *iₜₒₜ* et *i_{DC}* sont des constantes, il ressort que l'amplitude de modulation de l'intensité du battement électromagnétique est directement proportionnelle à la racine carrée de la réflectance *R* et que la composante continue de l'intensité du battement électromagnétique est proportionnelle à la réflectance *R.*

D'autres variantes et améliorations peuvent être envisagées sans pour autant sortir du cadre de l'invention tel que défini par les revendications ci-après.

### Liste des documents cités

[1] Hasan Ayaz et al. : "Optical imaging and spectroscopy for the study of the human brain: status report", Neurophoton, Vol. 9, No. S2, S24001, 30 août 2022, https://doi.org/10.1117/1.NPh.9.S2.S24001

## Revendications

1. Procédé de mesure, par spectroscopie par réflectance diffuse, du coefficient d'atténuation d'une partie d'un corps (1) diffusant et/ou absorbant, le procédé comprenant les étapes suivantes :
a) émission d'un rayonnement optique dont la fréquence optique est modulée, au moins une partie du rayonnement optique, dénommée signal de sonde, irradiant le corps,
b) réception d'une partie du signal de sonde diffusée et réfléchie par le corps, dénommée signal rétrodiffusé, et mesure de la longueur de parcours *d* du signal rétrodiffusé,
c) mesure de la réflectance *R* de la partie du corps traversée par le signal rétrodiffusé,
d) calcul, par une unité de traitement de données (25), du coefficient d'atténuation µ à partir de la longueur de parcours *d* et de la réflectance *R* mesurées,
l'étape a) comprenant la modulation de la fréquence optique du rayonnement optique selon une rampe de hauteur *h* et de durée *t,* et, comprenant la séparation du rayonnement optique en le signal de sonde et en un oscillateur local, l'oscillateur local n'irradiant pas le corps,
la mesure de la réflectance *R* comprenant le mélange du signal rétrodiffusé et de l'oscillateur local pour former un battement électromagnétique suivi du calcul, par l'unité de traitement de données (25), de la réflectance *R* à partir de l'amplitude de modulation de l'intensité du battement électromagnétique et/ou à partir de la valeur de la partie continue de l'intensité du battement électromagnétique.

2. Procédé selon la revendication précédente, le calcul du coefficient d'atténuation *µ* étant réalisé à partir de la formule suivante : $R = \frac{{e}^{- μ d}}{{d}^{2}} .$

3. Procédé selon l'une des revendications précédentes, le rayonnement optique étant modulé sinusoïdalement en intensité.

4. Procédé selon la revendication précédente, la mesure de la longueur de parcours *d* comprenant la mesure du déphasage *α* entre le signal rétrodiffusé et le rayonnement optique émis, suivie du calcul de la longueur de parcours *d* à partir de la formule suivante : $d = c . \frac{α}{4 . π . {f}_{mod}}$ où c est la vitesse de la lumière dans le vide et *f_{mod}* est la fréquence de modulation du rayonnement optique.

5. Procédé selon l'une des revendications 1 à 3, pour mesurer la longueur de parcours *d,* l'étape a) comprend la modulation de la fréquence optique du rayonnement optique selon une rampe de hauteur *h* et de durée *t,* et,
l'étape a) comprend la séparation du rayonnement optique en le signal de sonde et en un oscillateur local, l'oscillateur local n'irradiant pas le corps,
l'étape b) comprend le mélange du signal rétrodiffusé et de l'oscillateur local pour former un battement électromagnétique suivi du calcul de la longueur de parcours *d* à partir de la formule suivante : ${f}_{bat} = h . \frac{d}{c . t}$
où *f_{bat}* est la fréquence moyenne du battement électromagnétique,
de préférence la fréquence *f_{bat}* du battement électromagnétique est obtenue par un traitement comportant une transformée de Fourier de l'intensité du battement électromagnétique.

6. Dispositif (12) pour réaliser le procédé selon la revendication 5, le dispositif comprenant :
- une source d'émission lumineuse (15) configurée pour émettre un rayonnement optique cohérent, modulé en intensité avec une fréquence optique modulée selon une rampe de hauteur *h* et de durée *t,*
- un séparateur de faisceau (50) configuré pour séparer le rayonnement optique en un signal de sonde destiné à irradier un corps (1) diffusant et/ou absorbant et un oscillateur local (75),
- une unité de détection optique (55) comportant un multiplexeur (60) pour mélanger l'oscillateur local avec une partie du signal de sonde diffusée et réfléchie par le corps, dénommée signal rétrodiffusé, afin de former un battement électromagnétique, l'unité de détection optique comportant également au moins un récepteur optique (65) configuré pour mesurer l'intensité au cours du temps du battement électromagnétique ou également pour mesurer l'intensité moyenne du signal rétrodiffusé, la source d'émission lumineuse le séparateur de faisceau et l'unité de détection optique étant agencés pour être disposés d'un même côté du corps,
- une unité de traitement de données (25) configurée pour déterminer la fréquence moyenne *f_{bat}* du battement électromagnétique à partir de son intensité mesurée et pour calculer la longueur de parcours *d* à partir de la formule suivante : ${f}_{bat} = h . \frac{d}{c . t}$ et pour calculer le coefficient d'atténuation *µ* à partir la longueur de parcours *d* calculée et de la réflectance *R* de la partie du corps traversée par le signal rétrodiffusé mesurée à partir de l'intensité du battement électromagnétique.

7. Dispositif selon la revendication 6, comprenant un guide d'onde (70) pour conduire l'oscillateur local du séparateur de faisceau jusqu'à l'unité de détection optique.

8. Dispositif selon la revendication 6 ou 7, le récepteur optique comprenant au moins une photodiode à jonction PIN (80), de préférence deux photodiodes à jonction PIN connectées en séries.

9. Dispositif selon l'une des revendications 6 à 8, l'unité de traitement de données étant configurée de sorte que le calcul du coefficient d'atténuation *µ* est réalisé à partir de la formule suivante : $R = \frac{{e}^{- μ d}}{{d}^{2}} .$

10. Dispositif selon l'une des revendications 6 à 9, la source d'émission lumineuse étant configurée pour émettre un signal de sonde supplémentaire de même longueur d'onde que le rayonnement optique, d'intensité constante et destiné à irradier le corps
